(19) Europäisches Patentamt
European Patent Office
Office européen des brevets

(11) Veröffentlichungsnummer: **0 267 427 B1**

(12) # EUROPÄISCHE PATENTSCHRIFT

(45) Veröffentlichungstag der Patentschrift: **18.03.92**

(51) Int. Cl.[5]: **C07D 501/46**

(21) Anmeldenummer: **87114592.6**

(22) Anmeldetag: **06.10.87**

(54) **Neues Verfahren zur Herstellung von Cephalosporinderivaten.**

(30) Priorität: **07.10.86 AT 2657/86**

(43) Veröffentlichungstag der Anmeldung:
**18.05.88 Patentblatt 88/20**

(45) Bekanntmachung des Hinweises auf die Patenterteilung:
**18.03.92 Patentblatt 92/12**

(84) Benannte Vertragsstaaten:
**AT BE CH DE ES FR GB GR IT LI LU NL SE**

(56) Entgegenhaltungen:
**EP-A- 0 157 538**
**EP-A- 0 179 546**
**EP-A- 0 187 450**
**FR-A- 2 466 467**

(73) Patentinhaber: **BIOCHEMIE Gesellschaft m.b.H.**

**A-6250 Kundl Tirol(AT)**

(72) Erfinder: **Prager, Bernhard Christian**
**Steinbacherstrasse 1**
**A-6300 Wörgl(AT)**

(74) Vertreter: **Kleine-Deters, Johannes et al**
**Sandoz AG Patentabteilung**
**CH-4002 Basel(CH)**

EP 0 267 427 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft ein verbessertes, vereinfachtes und neues Verfahren zur Herstellung des Antibiotikums Ceftazidime.Pentahydrat.

Ceftazidime ist ein wertvolles Antibiotikum. Unter anderem wird seine Herstellung in der GB-PS 2025 398 beschrieben. Ceftazidime kann in verschiedenen Formen erhalten werden, etwa als Dihydrochlorid, Dihydrobromid oder als Pentahydrat. Das Pentahydrat ist die bevorzugte Form und wird beispielsweise in der GB-PS 2063 871 beschrieben. Die bekannten Verfahren, die zu dieser bevorzugten Form des Pentahydrats führen, verlaufen über den im folgenden Formelschema skizzierten Weg:

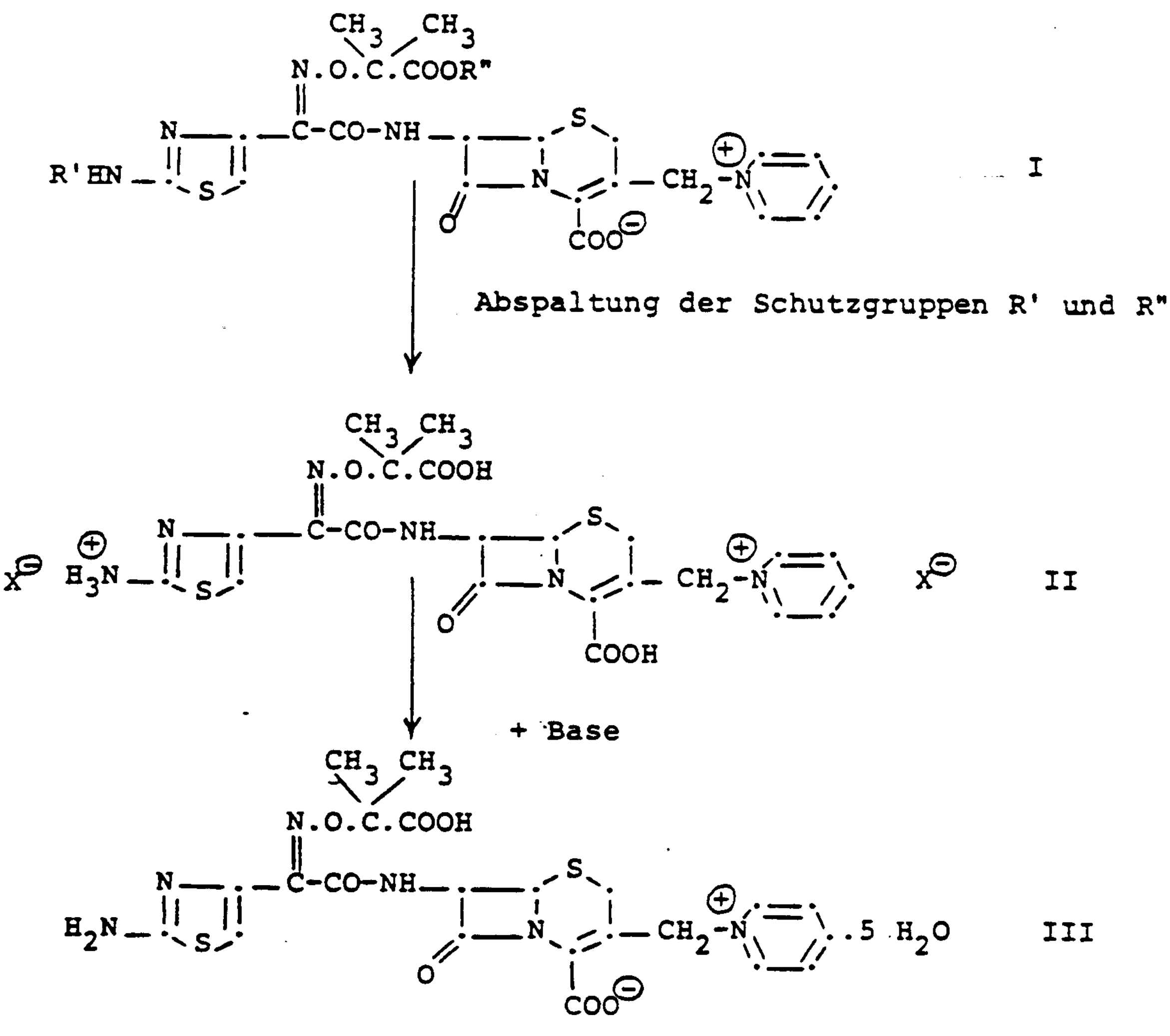


I

II

III

Bei den literaturbekannten Herstellverfahren des Ceftazidime.Pentahydrats (Formel III) werden aus Verbindungen der Formel I, welche das einfach (R' = Wasserstoff) oder doppelt geschützte (R' = Schutzgruppe) Ceftazidime darstellen, zuerst die Verbindungen der Formel II, welche das Ceftazidime als Dihydrohalogenid darstellen, hergestellt und isoliert (GB 2063871), oder man erzielt die Reinigung des Ceftazidimes von den Spaltprodukten durch Extraktionsprozesse (EPA 179546), bevor das Ceftazidime als Pentahydrat isoliert werden kann. Diese Zwischenisolierung des Dihalogenids oder die Reinigung der das Ceftazidime enthaltenden Lösung nach der Abspaltung der Schutzgruppen ist nötig, da die bei der Abspaltung der Schutzgruppen auftretenden Spaltprodukte die Kristallisation des reinen Pentahydrats verhindern bzw. zumindest erheblich erschweren. Zusätzlich wäre aufgrund des Einsatzes von erheblichen Mengen an organischen Säuren zur Abspaltung der Schutzgruppen (meist Ameisensäure) bei einer direkten Isolierung des Ceftazidimes als Pentahydrat ohne Zwischenisolierung des Ceftazidimes als Dihydrohalogenid eine erhebliche Salzbelastung bei der Ausfällung des Pentahydrats, welche bei einem pH-Wert von etwa 3,6 bis 4 erfolgt, gegeben, welche wiederum eine weitgehende Kristallisation des Ceftazidimes als Pentahydrat verhindert.

Erfindungsgemäß wurde nun überraschenderweise gefunden, daß es, ausgehend von der Verbindung der Formel I, worin R' = H und R" = t-Butyl darstellt, gelingt, direkt das Pentahydrat des Ceftazidimes zu kristallisieren, ohne daß man über die isolierte Zwischenstufe eines Dihalogenids gehen muß. Das erfindungsgemäße Verfahren ist dadurch gekennzeichnet, daß man bei der Abspaltung der Schutzgruppe R" = t-Butyl nur Salzsäure verwendet.

Während bei den bekannten Prozessen neben der Verwendung von Salzsäure immer weitere (organische) Säuren, die zumeist auch als Lösungsmittel dienen, wie etwa Ameisensäure, Dichloressigsäure, Trifluoressigsäure oder Trichloressigsäure, eingesetzt werden, beschränkt sich das erfindungsgemäße Verfahren auf die alleinige Verwendung von Salzsäure. Durch den Wegfall der anderen (meist organischen) Säuren wird ein erheblicher Vorteil insofern erzielt, als bei einer direkten Aufarbeitung zum Pentahydrat, welches bei etwa pH 3,6 bis 4 isoliert wird, nur die verwendete Salzsäure neutralisiert werden muß, während im Falle der zusätzlichen Verwendung (oder auch alleinigen Verwendung) einer organischen Säure im großen Überschuß, wie es bei den herkömmlichen Verfahren üblich ist, bei einer direkten Neutralisation ein gewaltiger Salzanteil anfällt, der eine Kristallisation des Pentahydrats des Ceftazidimes sehr schwer möglich macht.

Ein weiterer Vorteil des erfindungsgemäßen Verfahrens stellt die hohe Konzentration, mit der das Verfahren durchgeführt wird, dar - womit große Mengen an Produkt in geringer Zeit und mit wenig Reaktorvolumen hergestellt werden können. Ein weiterer Vorteil beim erfindungsgemäßen Verfahren liegt darin, daß außer Salzsäure (und zur Neutralisation eine Base) keinerlei Chemikalien oder Lösungsmittel benötigt werden, was einen bedeutenden finanziellen und ökologischen Vorteil darstellt.

Neben all diesen Vorteilen des erfindungsgemäßen Verfahrens ist jedoch auch die Möglichkeit gegeben, die Zwischenstufe des Dihydrochlorids des Ceftazidimes (Formel II; X = Chlorid) zu isolieren, wobei auch dabei die Vorteile hinsichtlich Ökologie und leichter Regenerierbarkeit der Lösungsmittel gegeben sind. Aus EPA 157538 ist bekannt, Ceftazidime Dihydrobromid direkt aus dem Reaktionsgemisch nach der Reaktion des doppelt geschützten (R' = Schutzgruppe) Ceftazidimes mit Ameisensäure/Bromwasserstoffsäure durch Zugabe eines mit Wasser mischbaren Lösungsmittels zur Kristallisation zu bringen.

Bei der Isolierung der Verbindung der Formel II wird beim erfindungsgemäßen Verfahren ein weiterer Vorteil deutlich, da aufgrund der alleinigen Verwendung von Salzsäure für die Esterspaltung die Vebindung der Formel II besonders leicht und in hochreiner Form gewonnen werden kann. Das erfindungsgemäße Verfahren erlaubt im Gegensatz zu den bekannten Verfahren - bei denen das Reaktionsgemisch meist mit riesigen Mengen Aceton gequenscht wird - eine einfache und langsame Kristallisation des Dihydrochlorids des Ceftazidimes durch einfachen Zusatz von Lösungsmitteln wie etwa Aceton oder/und Ethanol zum Reaktionsgemisch, wodurch des Dihydrochlorid des Ceftazidimes in hochreiner Form in gut isolierbaren Kristallen gewonnen wird. Das auf diese Weise isolierte Dihydrochlorid zeichnet sich durch gute Reinheit (HPLC ≧93 %) aus, was bei der Umwandlung in das Pentahydrat des Ceftazidimes erhebliche Vorteile bringt (erhöhte Kristallisationsausbeuten).

Die Mutterlauge bei der Isolierung des Dihydrochlorids, da sie neben Aceton und Salzsäure und geringen Mengen Iso-Buten keinerlei Lösungsmittel enthält, kann auf einfache Weise regeneriert bzw. entsorgt werden. Dazu genügt es, die Mutterlauge mit Hilfe von (gegebenenfalls fester) Alkalilauge zu neutralisieren (zur Vermeidung von Korrosion bei der Destillation) und das Aceton abzudestillieren bzw. zu rektifizieren. Das so erhaltene Aceton kann dann wieder für die Fällung der Verbindung der Formel II eingesetzt werden.

Das erfindungsgemäße Verfahren, das sich anstelle der sonst üblichen organischen Säuren bzw. Mischungen von organischen Säuren mit einer Halogenwasserstoffsäure auf die alleinige Verwendung von wäßriger Salzsäure für die Spaltung von Verbindungen der Formel I beschränkt, erlaubt also in einfacher Weise sowohl die Herstellung der als Zwischenstufe anzusehenden Verbindung der Formel II (X = Chlorid) wie auch die direkte Gewinnung des Pentahydrates des Ceftazidimes (Formel III) in hochreiner Form (≧97 % auf wasserfreier Basis) und ausgezeichneter Ausbeute (70 % und mehr).

Beim erfindungsgemäßen Verfahren kann der t-Butylester des Ceftazidimes in beliebiger Kristallform oder auch als Säureadditionssalz eingesetzt werden.

Die folgenden Beispiele sollen die Erfindung näher erläutern, diese jedoch keinesfalls einschränken. Alle Temperaturangaben erfolgen in Centigraden, die Wesserbestimmungen erfolgten mit der Karl-Fischer-Methode.

Beispiel 1: Ceftazidime.Pentahydrat

65 g Ceftazidime-t-butylester (kristallin in $\beta$-Form) werden bei 0 bis 5° in 80 ml HCl konz. ( 11 normal)

eingetragen, wobei eine hellgelbe Lösung entsteht. Diese wird noch 1 Stunde bei 5° gerührt, bis die Esterspaltung vollständig ist (DC- oder HPLC-Kontrolle). Dann wird die Lösung mit 80 ml Eiswasser versetzt und unter guter Kühlung der pH-Wert mit 5 N NaOH bei max. +5° auf 4,1 eingestellt. Es wird 1 Stunde bei 5 bis 10° stehen gelassen, wobei das Produkt auszukristallisieren beginnt. Dann wird der pH-Wert mit 3 N HCl auf 3,8 gestellt und nach einer weiteren Stunde auf pH 3,6, wobei die Temperatur immer zwischen +5 und +10° gehalten wird. Schließlich wird noch 5 Stunden auf 0 bis 4° gekühlt. Die ausgefallenen Kristalle werden dann abfiltriert, mit kaltem Wasser und Aceton gewaschen und bei Raumtemperatur getrocknet. Man erhält dabei 46,5 g der Titelverbindung (73 % d.Th.) in Form eines weißen kristallinen Pulvers. Gehalt (HPLC; auf $H_2O$-freier Basis): 97,0%
Wassergehalt (Karl-Fischer): 14,5%

Beispiel 2: Ceftazidime.Pentahydrat

70,2 g Ceftazidime-t-butylester (kristallin in α-Form) werden bei 0 bis 5° in 100 ml HCl konz. eingetragen, wobei eine hellgelbgrünliche Lösung entsteht. Diese wird noch etwa 1 Stunde bei 5° gerührt. Dann werden 120 ml Eiswasser zugesetzt und danach mit festem Natriumbikarbonat versetzt, bis ein pH-Wert von 4,0 - 4,1 erreicht ist. Die Temperatur wird dabei unter +5° gehalten. Man läßt dann 1 bis 2 Stunden bei etwa 10 bis 15° stehen, wobei das Produkt auszukristallisieren beginnt. Dann wird mit 3 N HCl der pH-Wert auf 3,6 eingestellt und noch 5 Stunden bei 0 bis 4° stehen gelassen. Dann wird die Titelverbindung isoliert, mit kaltem Wasser und Aceton gewaschen und getrocknet. Man erhält auf diese Weise 44,3 g (70 %) der Titelverbindung in reiner Form (HPLC-Gehalt: 97,3 %, $H_2O$-Gehalt:14,6 %).

Beispiel 3: Ceftazidime.Dihydrochlorid

130 g Ceftazidime-t-butylester (kristallin in β-Form) werden bei 10° in 150 ml HCl konz. eingetragen, wobei eine gelbe Lösung entsteht. Diese wird noch eine Stunde bei 10° gerührt. Dann wird mit 300 ml Aceton versetzt und 30 Minuten kristallisieren gelassen. Das Produkt scheidet sich dabei in Form schöner gleichmäßiger Kristalle ab. Zur Vervollständigung der Kristallisation werden im Verlauf von 90 Minuten noch 700 ml Aceton zugesetzt und dann noch eine Stunde gerührt. Die Kristalle werden anschließend isoliert, mit Aceton gewaschen und im Vakuum getrocknet. Man erhält dabei 120 g (97 %) der Titelverbindung in Form von weißen rieselfähigen Kristallen mit einem Gehalt von 94 % (HPLC).

Beispiel 4: Ceftazidime.Pentahydrat

60 g des, wie in Beispiel 3 beschrieben, erhaltenen Ceftazidime.Dihydrochlorids werden in 300 ml Eiswasser gelöst. Die saure (pH 0,7 - 0,8) Lösung wird zur Entfernung von Fremdpartikeln blankfiltriert und dann bei maximal 10° mit etwa 160 ml 3 N NaOH auf pH 4,0 gestellt. Nach 2 Stunden Kristallisationsdauer wird der pH-Wert auf 3,6 eingestellt und weitere 3 Stunden bei etwa 5° kristallisieren gelassen. Das Produkt wird dann abgenutscht, mit kaltem Wasser und Aceton gewaschen und getrocknet. Man erhält dabei 50 g (87 %) der Titelverbindung in Form von farblosen Kristallen mit einem Gehalt von ≧ 97 %.

**Patentansprüche**

**1.** Neues Verfahren zur Herstellung des Pentahydrats des Ceftazidimes der Formel

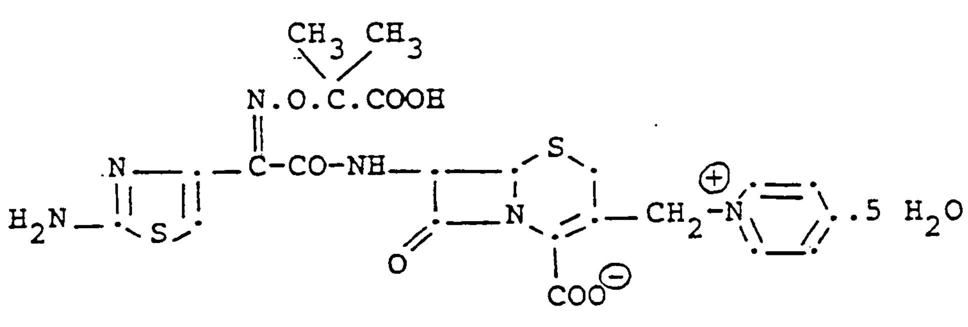

III

dadurch gekennzeichnet, daß man die Estergruppe der Verbindung der Formel

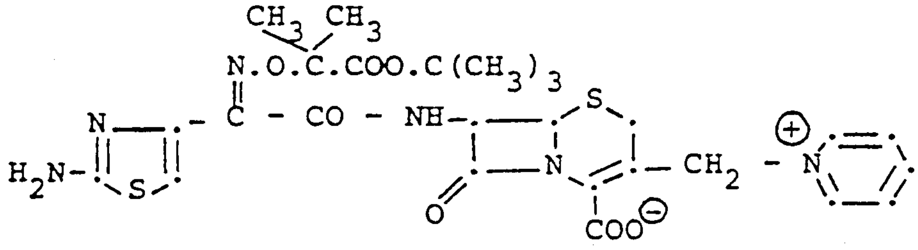

Ia

oder ein Säureadditionssalz davon unter Verwendung von wässriger konzentrierter Salzsäure spaltet und das dabei entstehende Ceftazidime entweder direkt aus dem Reaktionsgemisch durch Basenzusatz bei einem pH-Wert von 3,6 bis 4,1 als Pentahydrat kristallisiert oder zuerst durch Zusatz von Aceton oder/und Ethanol in an sich bekannter Weise das Dihydrochlorid des Ceftazidimes isoliert und dieses nach an sich bekannter Methode ins Pentahydrat überführt.

**2.** Verfahren nach Anspruch 1, dadurch gekennzeichnet, daß die Konzentration der wäßrigen Salzsäure 20 bis 40 Gew.% beträgt.

**3.** Verfahren nach den Ansprüchen 1 und 2, dadurch gekennzeichnet, daß die Esterspaltung bei -15 bis +35° C durchgeführt wird.

**4.** Verfahren nach den Ansprüchen 1 bis 3, dadurch gekennzeichnet, daß ohne Zwischenisolierung des Dihydrochlorids des Ceftazidimes direkt das Pentahydrat des Ceftazidimes aus der Reaktionslösung kristallisiert wird.

**5.** Verfahren nach den Ansprüchen 1 bis 3, dadurch gekennzeichnet, daß das Dihydrochlorid des Ceftazidimes aus dem Reaktionsgemisch durch Zusatz von Aceton oder/und Ethanol kristallisiert und dieses nach an sich bekannter Methode ins Pentahydrat des Ceftazidimes überführt wird.

**6.** Verfahren zur Herstellung des Dihydrochlorids des Ceftazidimes dadurch gekennzeichnet, dass man die Estergruppe der Verbindung der Formel Ia, wie im Anspruch 1 dargestellt oder ein Säureadditionssalz davon unter Verwendung von wässriger Salzsäure spaltet und das darausresultierende Dihydrochlorid durch Zusatz von Aceton und/oder Ethanol in an sich bekannter Weise kristallisiert.

## Claims

**1.** New process for the preparation of the pentahydrate of ceftazidime of formula

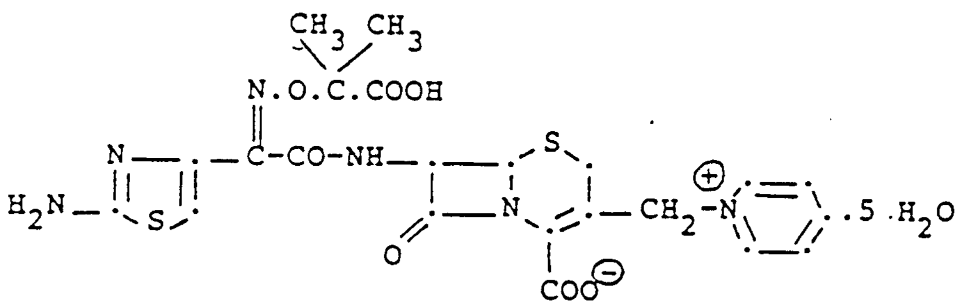

III

characterised by cleaving the ester group of the compound of formula

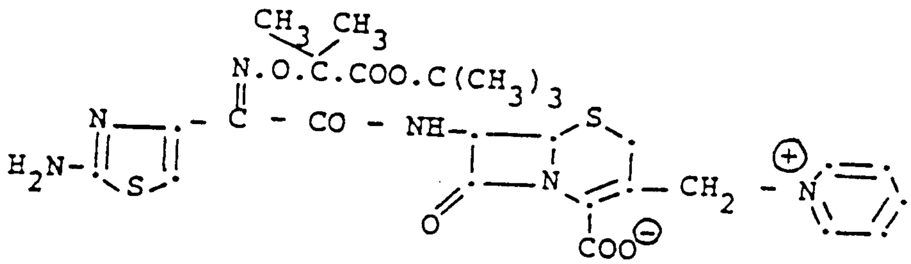

Ia

or an acid addition salt thereof using aqueous concentrated hydrochloric acid, and the ceftazidime thus obtained either crystallising directly from the reaction mixture as the pentahydrate at a pH value from 3.6 to 4.1 by adding a base, or at first isolating the dihydrochloride of the ceftazidime in known manner by adding acetone and/or ethanol and converting this into the pentahydrate by known methods.

**2.** Process according to claim 1, characterised in that the concentration of the aqueous hydrochloric acid is 20 to 40% by weight.

**3.** Process according to claims 1 and 2, characterised in that the ester cleavage is carried out at -15 to +35 °C.

**4.** Process according to claims 1 to 3, characterised in that the pentahydrate of ceftazidime is crystallised from the reaction solution directly without intermediate isolation of the ceftazidime dihydrochloride.

**5.** Process according to claims 1 to 3, characterised in that the dihydrochloride of ceftazidime is crystallised from the reaction mixture by adding acetone and/or ethanol, and this is converted into the ceftazidime pentahydrate by known methods.

**6.** Process for the preparation of the dihydrochloride of ceftazidime, characterised in that the ester group of the compound of formula Ia, as illustrated in claim 1, or an acid addition salt thereof, is cleaved using aqueous hydrochloric acid, and the so obtained dihydrochloride is crystallised in known manner by adding acetone and/or ethanol.

## Revendications

**1.** Nouveau procédé de préparation du pentahydrate de ceftazidime de formule

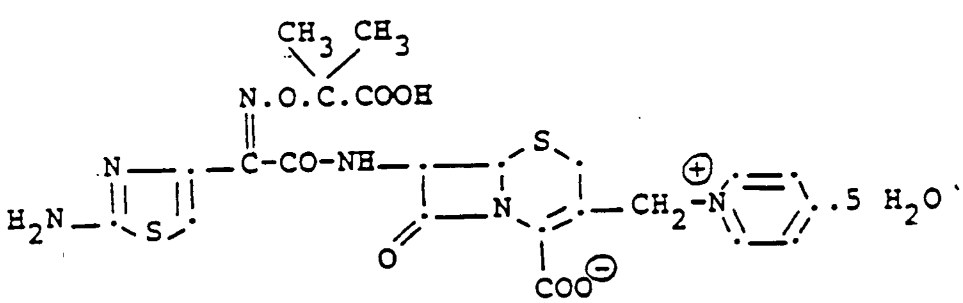

III

caractérisé en ce qu'on scinde le groupe ester du composé de formule

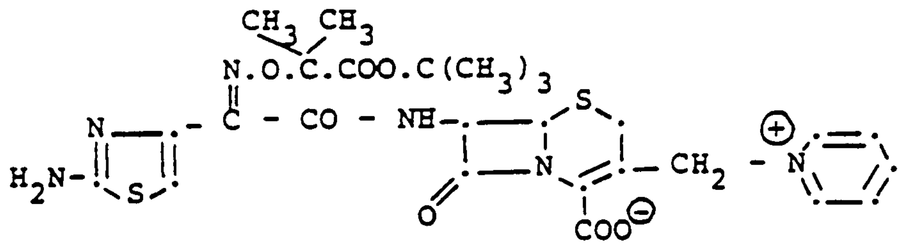

Ia

ou d'un sel d'addition d'acide de ce composé en utilisant de l'acide chlorhydrique concentré aqueux et soit on cristallise sous forme de pentahydrate directement à partir du mélange réactionnel, à un pH de 3,6 à 4,1 par addition d'une base, la ceftazidime qui se forme, soit on isole d'abord le dichlorhydrate de ceftazidime de manière connue en soi par addition d'acétone et/ou d'éthanol et on le transforme selon les méthodes connues en pentahydrate.

**2.** Procédé selon la revendication 1, caractérisé en ce que la concentration de l'acide chlorhydrique aqueux est de 20 à 40% en poids.

**3.** Procédé selon la revendication 1 ou 2, caractérisé en ce que la scission de l'ester est effectuée à une température de -15 à +35 ° C.

**4.** Procédé selon les revendications 1 à 3, caractérisé en ce qu'on cristallise directement le pentahydrate de ceftazidime à partir de la solution de réaction, sans isolement intermédiaire du dichlorhydrate de ceftazidime.

**5.** Procédé selon les revendications 1 à 3, caractérisé en ce qu'on cristallise le dichlorhydrate de ceftazidime à partir du mélange réactionnel par addition d'acétone et/ou d'éthanol et on le transforme selon les méthodes connues en pentahydrate de ceftazidime.

**6.** Procédé de préparation du dichlorhydrate de ceftazidime, caractérisé en ce qu'on scinde le groupe ester du composé de formule Ia, telle que représentée à la revendication 1, ou d'un sel d'addition d'acide de ce composé en utilisant de l'acide chlorhydrique aqueux et on cristallise le dichlorhydrate résultant de manière connue en soi par addition d'acétone et/ou d'éthanol.